# EUROPEAN PATENT APPLICATION

(11) **EP 2 011 493 A1**
(43) Date of publication of application: **07.01.2009**
(21) Application number: 07737022.9
(22) Date of filing: 04.04.2007
(51) Int. Cl.: A61K 31/22, A61K 31/23, A61P 3/04, A61P 3/10

(54) **INHIBITOR OF INCREASE IN POSTPRANDIAL BLOOD INSULIN LEVEL**

(30) Priority: 24.04.2006 JP 2006119049
(71) Applicant: KAO CORPORATION, Chuo-ku Tokyo 103-8210 (JP)
(72) Inventor: TAKENO, Nanami, Ina-shi, Nagano 3960021 (JP); SHIMOTOYODOME, Akira, Ichikai-machi, Haga-gun, Tochigi 321-3497 (JP); MEGURO, Shinichi, Sumida-ku, Tokyo 1318501 (JP)
(74) Representative: Vossius & Partner
(86) International application number: PCT/JP2007/000365
(87) International publication number: WO 2007/129439

(57) **Abstract**

The present invention provides an agent for inhibiting a postprandial increase in blood insulin level, wherein the agent containing a monoacylglycerol as an active ingredient. An agent of the invention for inhibiting a postprandial increase in blood insulin level contains a monoacylglycerol as an active ingredient.

## Description

### Technical Field

The present invention relates to an agent for inhibiting a postprandial increase in blood insulin level, which is useful as a drug or food ingredient.

### Background Art

When blood glucose level increases after eating, insulin is secreted from pancreatic β-cells in the pancreas, which promotes uptake of glucose into adipose tissue and muscle tissue, thereby promoting synthesis of fat in the liver and muscle, and suppressing decomposition and burning of fat.
However, when insulin secretion continues in a hyperglycemic state, insulin sensitivity is reduced (i.e., insulin resistance increases) in insulin target organs such as skeletal muscle, liver, and adipose tissue, and thus a larger amount of insulin is secreted from the pancreas. Repetition of such insulin secretion finally leads to exhaustion of the pancreas, and reduction in insulin secretion from pancreatic β-cells. However, insulin target organs remain in a state of increased insulin resistance. As has been known, such malfunction of the action mechanism of insulin may result in a predisposition to, for example, obesity or diabetes, and eventually in, for example, obesity or type II diabetes (hyperglycemia).
Regarding prevention or amelioration of obesity, diabetes (hyperglycemia), etc. associated with modern lifestyles (overeating, high-fat diets, and lack of exercise), attention has been paid to blood glucose level, and, among others, postprandial blood glucose level has generally been measured as an important item. However, virtually little attention has been paid to postprandial blood insulin level.

Monoacylglycerol (hereinafter may be abbreviated as "MAG") is a substance which exhibits high safety and is widely used as, for example, an emulsifier in the field of food. Generally, monoacylglycerol is incorporated into, for example, margarine, milk beverage, ice cream, or bread in an amount of about 0.2 to about 0.5% (Non-Patent Documents 1 and 2). As has been known, monoacylglycerol has the effect in inhibiting a postprandial increase in blood triglyceride level (Patent Document 1). However, the relationship between monoacylglycerol and postprandial blood insulin level has not yet been determined.
Patent Document 1: JP-A-1993-310567
Non-Patent Document 1: Kazuhiro Okamura, et al., Shokuhin Tenkabutsu no Siyoho ("Method for Use of Food Additive"), 1973, Shokuhin to Kagakusha, pp. 288-289
Non-Patent Document 2: Seiji Fujii, et al., Syokuhin Tenkabutsu Handobukku ("Food Additive Handbook"), 1997, Koseikan Co., Ltd., pp. 236-238

### Disclosure of the Invention

The present invention provides the following inventions (1) to (8).
(1) An agent for inhibiting a postprandial increase in blood insulin level, wherein the agent containing a monoacylglycerol as an active ingredient.
(2) An agent for inhibiting a postprandial increase in blood insulin level as described in claim 1, which inhibits an excessive postprandial increase in blood insulin level.
(3) A method for inhibiting a postprandial increase in blood insulin level, including causing a subject in need thereof to consume a monoacylglycerol, or administering a monoacylglycerol to a subject in need thereof.
(4) A method for preventing or ameliorating diabetes, including causing a subject in need thereof to consume a monoacylglycerol, or administering a monoacylglycerol to a subject in need thereof.
(5) A method for preventing or ameliorating obesity, including causing a subject in need thereof to consume a monoacylglycerol, or administering a monoacylglycerol to a subject in need thereof.
(6) Use of a monoacylglycerol as an agent for inhibiting a postprandial increase in blood insulin level.
(7) Use of a monoacylglycerol as an agent for preventing or ameliorating diabetes.
(8) Use of a monoacylglycerol as an agent for preventing or ameliorating obesity.

### Detailed Description of the Invention

The present invention is directed to an agent for inhibiting a postprandial increase in blood insulin level, which is useful as a drug or food ingredient.

The present inventors have found that when a monoacylglycerol is administered to mice fed with a diet containing carbohydrate and lipid, an excessive postprandial increase in blood insulin level is inhibited as compared with the case where no monoacylglycerol is administered.

According to the present invention, a postprandial increase in blood insulin level can be inhibited so that the level falls within a proper range. Therefore, the present invention can ameliorate or prevent problems associated with secretion of a large amount of insulin, such as exhaustion of pancreatic β-cells and increased insulin resistance of insulin target organs. Thus, the present invention can prevent diabetes (hyperglycemia) or obesity, or prevent individuals from becoming candidates therefor, and as well can improve an individual's physical condition.

Examples of the monoacylglycerol employed in the present invention include a monoacylglycerol obtained through esterification of the hydroxyl group at 1-position of glycerin with a fatty acid (1-monoacylglycerol); a monoacylglycerol obtained through esterification of the hydroxyl group at 2-position of glycerin with a fatty acid (2-monoacylglycerol); and a monoacylglycerol obtained through esterification of the hydroxyl group at 3-position of glycerin with a fatty acid (3-monoacylglycerol). Preferably, a 1-monoacylglycerol is employed.
No particular limitation is imposed on the number of carbon atoms of the fatty acid residue, but the number is preferably 8 to 24, more preferably 16 to 22. Examples of the fatty acid residue include saturated fatty acid residues and unsaturated fatty acid residues. Specific examples include acyl groups derived from caprylic acid, capric acid, lauric acid, myristic acid, palmitic acid, stearic acid, oleic acid, linoleic acid, linolenic acid, eicosapentaenoic acid and docosahexaenoic acid; acyl groups derived from a mixture of the aforementioned fatty acids; and acyl groups derived from fatty acids from oils containing the aforementioned fatty acids (e.g., animal oils such as beef tallow and lard, and vegetable oils such as palm oil, rapeseed oil, soybean oil, safflower oil, corn oil, shiso oil, stillingia oil, linseed oil, and perilla oil). These monoacylglycerols may be employed singly or in combination of two or more species.

When these monoacylglycerols are employed in combination, the ratio of the total amount of the unsaturated fatty acid residues to the total amount of the entire fatty acid residues is preferably 55% or more, more preferably 70% or more, still more preferably 90% or more. Yet still more preferably, the unsaturated fatty acid residues include oleic acid residues in an amount of 50 to 100%. The amount of fatty acid residues constituting the monoacylglycerol is estimated by converting the amount of acyl group constituting the monoacylglycerol to the amount of fatty acids.

The monoacylglycerol employed in the present invention may be produced through any reaction; for example, hydrolysis of an oil containing an unsaturated acyl group, such as linseed oil, perilla oil, shiso oil, soybean oil, or rapeseed oil; transesterification of glycerin with the oil as described above; or esterification of glycerin with a fatty acid derived from the oil as described above. The reaction may be carried out through a chemical technique employing an alkali catalyst or a similar catalyst, or through a biochemical technique employing an enzyme such as lipase. The resultant reaction product may be subjected to fractionation for isolation of a desired monoacylglycerol.

As described in the Example hereinbelow, when monoacylglycerol is consumed together with triacylglycerol (hereinafter may be abbreviated as "TAG"), monoacylglycerol exhibits the effect in significantly inhibiting an increase in blood insulin level caused by consumption of glucose or triacylglycerol. Therefore, monoacylglycerol can be employed as an agent for inhibiting a postprandial increase in blood insulin level in a food or drug for human or animals, or as an ingredient for such a food or drug. As used herein, "inhibition of a postprandial increase in blood insulin level" refers to inhibition of an excessive increase in blood insulin level caused by consumption of a diet containing lipid and carbohydrate; preferably, a diet containing a large amount of lipid, more preferably, a diet containing a large amount of triacylglycerol. As used herein, "excessive increase in blood insulin level" means that postprandial blood insulin level is higher than that as measured for the baseline case where only carbohydrate is ingested.

Regarding the agent for inhibiting a postprandial increase in blood insulin level of the present invention, the monoacylglycerol of the present invention may be administered singly to a human or an animal, or the monoacylglycerol may be incorporated into, for example, a food, a beverage, a drug, or a pet food to be consumed. The agent of the present invention may be applied to a food or beverage with a label indicating that it contains monoacylglycerol, exhibits the effect in inhibiting an excessive postprandial increase in blood insulin level, and is consumed for inhibiting an excessive postprandial increase in blood insulin level. The agent of the present invention may also be applied to a food or beverage such as food for beauty purposes, food for the sick, or food for specified health use with a label indicating that it is consumed for amelioration or prevention of diabetes (hyperglycemia), obesity, etc. When the agent of the present invention is employed in the form of a drug, the agent may be prepared into a solid dosage form for oral administration such as tablets or granules or a liquid dosage form for oral administration such as a liquid for internal use or a syrup.

A solid dosage form for oral administration such as tablets, coated tablets, granules, powder or capsules can be prepared by adding, to the monoacylglycerol of the present invention, an excipient and, if necessary, an additive such as a binder, a disintegrating agent, a lubricant, a coloring agent, a sweetening agent, or a flavoring agent, followed by customary processing. A liquid dosage form for oral administration such as a liquid for internal use, a syrup or an elixir can be prepared by adding, to the monoacylglycerol of the present invention, an additive such as a sweetening agent, a buffer, a stabilizer, or a sweetening agent, followed by customary processing.
The amount of the monoacylglycerol contained in any of the aforementioned dosage forms is generally 0.1 mass% or more, preferably 1 mass% or more, more preferably 5 wt.% or more, on the basis of the entire mass of the composition.

The effective amount of any of the aforementioned dosage forms which is administered to (consumed by) a subject in need thereof is preferably 0.01 to 10 g as reduced to monoacylglycerol per day. The agent for inhibiting a postprandial increase in blood insulin level of the present invention is effectively employed before, during, or after a meal.

### Examples

### Example 1 Effect of monoacylglycerol in inhibiting postprandial increase in blood insulin level in mouse

Triolein was employed as a triacylglycerol, and 1-monoolein was employed as a monoacylglycerol.
Male C57BL/6J mice (eight weeks old) were divided into groups (eight mice each). Through a probe, mice of the respective groups were orally administered the following substance: only glucose (2 mg/g-body weight) (Glucose); glucose (2 mg/g-body weight) plus triolein (TAG) (2 mg/g-body weight) emulsified with egg yolk lecithin (0.02 mg/g-body weight) (TAG/MAGO); TAG/MAG0 supplemented with 1-monoolein (MAG) of 0.08, 0.2 and 0.4 mg/g-body weight, respectively (TAG/MAG1, TAG/MAG2 and TAG/MAG3). Table 1 shows the compositions of the emulsions. Blood was collected from the orbital vein of each mouse over time until 30 minutes after oral administration, and blood insulin level was measured, followed by calculation of the area under the curve (AUC) of a graph. Blood insulin level was measured through ELISA (insulin assay kit, product of Morinaga Institute of Biological Science, Inc.).
Table 2 shows the blood insulin levels (AUCs) in mice 30 minutes after meal, which are values relative to the blood insulin level (AUC) of the mice administered only glucose that was taken as 100.

**[Table 1]**

| Composition of emulsion | Glucose (mg/g-body weight) | Triolein (mg/g-body weight) | 1-Monodein (mg/g-body weight) |
|---|---|---|---|
| Glucose | 2.0 | 0.0 | 0.0 |
| TAG/MAG0 | 2.0 | 2.0 | 0.0 |
| TAG/MAG1 | 2.0 | 2.0 | 0.08 |
| TAG/MAG2 | 2.0 | 2.0 | 0.2 |
| TAG/MAG3 | 2.0 | 2.0 | 0.4 |

| | | | |
|---|---|---|---|
| TAG/MAG0: glucose-supplemented TAG + MAG (0 mg) TAG/MAG1: glucose-supplemented TAG + MAG (0.08 mg) TAG/MAG2: glucose-supplemented TAG + MAG (0.2 mg) TAG/MAG3: glucose-supplemented TAG + MAG (0.4 mg) | | | |

**[Table 2]**

| Amount of postprandial insulin secretion in mice (AUC, relative value) | |
|---|---|
| Composition of emulsion | Blood insulin level (ng/mL) |
| Glucose | 100 ± 6.6 (N = 12) |
| TAG/MAG0 | 166.7 ± 15.7*** (N = 12) |
| TAG/MAG1 | 147.0 ± 12.8* (N = 14) |
| TAG/MAG2 | 115.0 ± 9.7## (N = 14) |
| TAG/MAG3 | 86.0 ± 8.2### (N = 14) |

| | |
|---|---|
| Statistically significant difference in blood insulin level between the Glucose group and the TAG/MAG0 or TAG/MAG1 group: ***: P<0.001, *: P<0.05 Statistically significant difference in blood insulin level between the TAG group and the TAG/MAG2 or TAG/MAG3 group: ###: P<0.001, ##: P<0.01 | |

As shown in Table 2, when the mice consumed TAG, postprandial blood insulin level (amount of postprandial insulin secretion) was increased as compared with the level in the mice consumed only glucose. However, the mice that received MAG in an amount of 1/10 or 1/5 of that of TAG exhibited a decreased postprandial blood insulin level as compared with the mice of the TAG/MAG0 group. The data indicate an inhibitory effect of MAG on postprandial increase in blood insulin level.

**[Table 3]**

| Formulation Example (1) Coffee beverage | |
|---|---|
| 1-MAG | 4.1 mass% |
| Coffee bean | 5.5 mass% |
| Milk | 7.0 mass% |
| Sugar | 6.0 mass% |
| Flavor | Slight amount |
| Sodium bicarbonate | (Adjusted to pH 6.5) |
| Water | Balance |

**[Table 4]**

| Formulation Example (2) Candy | |
|---|---|
| 1-MAG | 0.1 mass% |
| Sucrose ester (emulsifier) | 0.2 mass% |
| Thick malt syrup | 35 mass% |
| Sugar | 35 mass% |
| Flour | 5 mass% |
| Condensed milk | 17 mass% |
| Milk | 6 mass% |
| Butter | 2 mass% |
| Flavor | Appropriate amount |

## Claims

1. An agent for inhibiting a postprandial increase in blood insulin level, wherein the agent comprising a monoacylglycerol as an active ingredient.

2. An agent for inhibiting a postprandial increase in blood insulin level as described in claim 1, which inhibits an excessive postprandial increase in blood insulin level.

3. A method for inhibiting a postprandial increase in blood insulin level, comprising causing a subject in need thereof to consume a monoacylglycerol, or administering a monoacylglycerol to a subject in need thereof.

4. A method for preventing or ameliorating diabetes, comprising causing a subject in need thereof to consume a monoacylglycerol, or administering a monoacylglycerol to a subject in need thereof.

5. A method for preventing or ameliorating obesity, comprising causing a subject in need thereof to consume a monoacylglycerol, or administering a monoacylglycerol to a subject in need thereof.

6. Use of a monoacylglycerol as an agent for inhibiting a postprandial increase in blood insulin level.

7. Use of a monoacylglycerol as an agent for preventing or ameliorating diabetes.

8. Use of a monoacylglycerol as an agent for preventing or ameliorating obesity.
